# EUROPÄISCHE PATENTSCHRIFT

(11) **EP 0 923 959 B1**
(45) Veröffentlichungstag und Bekanntmachung des Hinweises auf die Patenterteilung: **21.05.2003**
(21) Anmeldenummer: 98120993.5
(22) Anmeldetag: 05.11.1998
(51) Int. Cl.: A61M 16/20, F16K 7/12

(54) **Ventil zum Regeln eines Gasflusses**
Gas flow control valve
Vanne pour la commande d'écoulement de gaz

(30) Priorität: 15.12.1997 SE 9704660
(43) Veröffentlichungstag der Anmeldung: 23.06.1999
(73) Patentinhaber: Siemens-Elema AB, 171 95 Solna (SE)
(72) Erfinder: Rydin, Göran, 183 50 Täby (SE); Eriksson, Per-Göran, 183 40 Täby (SE); Rydgren, Göran, 230 44 Bunkeflostrand (SE)

(56) Entgegenhaltungen:
- EP-A1- 0 444 007
- DE-A1- 3 342 482
- US-A- 5 265 594

## Beschreibung

Die Erfindung betrifft ein Ventil zum Regeln eines Gasflusses, umfassend ein Ventilgehäuse mit einem Einlaß für das Gas, das geregelt werden soll, und einem Auslaß für einen geregelten Gasfluß, einem Ventilsitz mit einer Ventilöffnung, einem bewegbaren Verschlußteil, umfassend eine steuerbare Welle, wobei die Endseite des Verschlußteils eine Dichtung, die zwischen dem Ventilsitz und dem Verschlußteil angeordnet ist, derart beeinflussen kann, daß das Verschlußteil die Ventilöffnung schließt und öffnet sowie den Gasfluß durch die Ventilöffnung regelt.

Ein Ventil dieser Art ist in der US-PS 5 265 594 gezeigt und beschrieben. Es ist in Verbindung mit Zufuhr von Gas zu einem Patienten, der an einem Beatmungsgerät angeschlossen ist, von großer Bedeutung, daß große wie kleine Gasmengen mit Hilfe eines solchen Ventils mit großer Genauigkeit und Wiederholbarkeit geregelt wird. Um zu versuchen, dies zu erreichen, ist es erforderlich, daß die Dichtung, die in der genannten US-Schrift eine Membarn ist, die am Ventilgehäuse festgespannt ist, sich beim Öffnen vom Ventilsitz parallel zu diesem hebt und beim Schließen, mit Hilfe des Wellenendes, mit einer gleichmäßig verteilten Kraft gegen den Ventilsitz drückt. Auch wenn die Membran mit Hilfe einer solchen Welle, die hier eine Solenoidenwelle ist, einen derartigen, scheinbar perfekten Bewegungsmuster folgt, können Probleme entstehen, insbesondere in Verbindung mit dem Öffnen der Ventilöffnung, wenn die Membran sich von dem Ventilsitz lösen soll. Es können z.B. Probleme in Verbindung mit Abnutzung oder in Verbindung mit Alterung der Membran und wenn die Membran in einem ersten Bewegungsmoment am Ventilsitz hängenbleibt und danach abrupt öffnet, entstehen, wobei eine genaue und wiederholte Steuerung des Flußes, insbesondere in Verbindung mit kleinen Mengen, schwer zu erreichen sein kann. Auch die Verzögerung oder die Hystereses, die bei der Magnetisierung der Solenoidenwelle beim Aktivieren derselben entstehen kann, kann eine unerwünschte Steuerung des Gasflußes in Verbindung mit dem Öffnen der Ventilöffnung mit den oben beschriebenen negativen Folgen verursachen.

Der Erfindung liegt die Aufgabe zugrunde, ein Ventil der Eingangs genannten Art zu schaffen, bei dem insbesondere kleine Gasflüße mit großer Genauigkeit und großer Dynamik wiederholt geregelt werden können.

Diese Aufgabe wird erfindungsgemäß gelöst, indem die Ebene der Endseite des Verschlußteils und die Ebene des Ventilsitzes einen Winkel bilden, wobei die Endseite des Verschlußteils derart gegen den Ventilsitz gedrückt bzw. von diesem gelöst werden kann, daß die Endseite die Ventilöffnung sukzessiv schließt bzw. öffnet. Durch die erwähnten Winkel wird im Moment des Öffnens bzw. des Schließens zwischen der Dichtung und dem Ventilsitz oder, wenn die Dichtung am Ventilsitz angebracht ist, zwischen der Dichtung und der Endseite des Verschlußteils immer ein Spalt gebildet, der sich über einen verhältnismäßig kleinen Teil der Anlagefläche der Dichtung bzw. des Ventilsitzes erstreckt, wobei nunmehr ein sukzessives Öffnen bzw. Schließen der Ventilöffnung erfolgt, ohne daß das Ventil abrupt geöffnet bzw. geschlossen wird. Hierdurch ist sichergestellt, daß sowohl große als auch kleine Gasflüsse mit großer Genauigkeit und mit großer Dynamik wiederholt geregelt werden können.

In einer vorteilhaften Ausführungsform des Ventils nach der Erfindung wird vorgeschlagen, daß die Ebene des Ventilsitzes schräg zur Zentrumachse des Ventilsitzes liegt. Bei einer solchen Ausführungsform liegt die Ebene der Endseite des Verschlußteils senkrecht zu dessen Zentrumachse.

In Verbindung mit einer weiteren Ausführungsform des Ventils nach der Erfindung wird vorgeschlagen, daß die Ebene der Endseite des Verschlußteils schräg zu dessen Zentrumachse liegt.

Nach der Erfindung kann der Winkel zwischen den genannten Ebenen zwischen 0,3° und 2°, vorzugsweise 0,7° sein. Der erwähnte Winkel wird vorzugsweise an die Größe der Ventilöffnung und an die Stärke der Dichtung angepasst.

In einer vorteilhaften Weiterbildung des Ventils nach der Erfindung wird vorgeschlagen, daß die Dichtung eine am Ventilgehäuse festgespannte Membran ist und daß die Membran mit einer steifen ebenen Platte versehen ist, die genau vor dem Ventilsitz angeordnet ist und die eine Flächengröße aufweist, die wenigstens die Flächengröße des Ventilsitzes hat und daß der Teil der Endseite des Verschlußteils, der die Membran beeinflußt, eine Flächengröße aufweist, die kleiner als die Flächengröße des Ventilsitzes ist. Aufgrund der Flächengröße desjenigen Teils der Endseite des Verschlußteils, der die Membran bzw. die Platte beeinflußt, kann beim Schließen und Öffnen der Ventilöffnung die steife Platte gegen die erwähnte Endseite kippen, wobei durch die Platte die Ventilöffnung sukzessiv geschlossen bzw. geöffnet werden kann. Die Platte kann vorzugsweise in der Membran integriert sein, kann aber auch an der Membran festgeklebt sein. Durch die Ausbildung der Membran kann die Ebene des Ventilsitzes, wenn es gewünscht wird, einen zur Zentrumachse verhältnismäßig großen Winkel aufweisen. Außerdem ist durch die Tatsache, daß die Platte gegen die Endseite des Verschlußteils kippen kann, auch bei einer eventuellen Deformierung der Membran immer ein sukzessives Öffnen und Schließen des Ventilsitzes gegeben. Die Endseite des Verschlußteils ist vorzugsweise abgerundet.

Die Aufgabe der Erfindung in Verbindung mit einem Ventil der Eingangs genannten Art kann vorzugsweise auch erreicht werden, indem zwischen der Endseite des Verschlußteils und dem Ventilsitz ein gegenüber dem Ventilsitz schräg angeordnetes, vorgeformtes Teil angebracht ist, das eine Flächengröße aufweist, die weningstens der Flächengröße des Ventilsitzes entspricht, wobei die Endseite des Verschlußteils so angeordnet ist, daß sie das schräg angeordnete, vorgeformte Teil derart gegen den Ventilsitz drückt bzw. vom Ventilsitz löst, daß das schräg angeordnete, vorgeformte Teil die Ventilöffnung sukzessiv schließt bzw. öffnet. Mit einem solchen schräg angeordenten, vorgeformten Teil werden die Vorteile, die in Verbindung mit den bereits beschriebenen Ebenen, die einen Winkel bilden, erwähnt sind, erhalten. Durch das schräg angeordnete, vorgeformte Teil braucht die Ebene der Endseite des Verschlußteils oder die Ebene des Ventilsitzes nicht im Verhältnis zu ihren Zentrumachsen abgewinkelt zu werden.

In einer vorteilhaften Weiterentwicklung des Ventils nach der Erfindung wird vorgeschlagen, daß der Teil der Endseite des Verschlußteils, der das schräg angeordnete Teil beeinflußt, eine Flächengröße aufweist, die kleiner als die Flächengröße des Ventilsitzes ist. Die Endseite ist vorzugsweise abgerundet ausgebildet. Hierduch ist auch bei einer eventuellen Deformierung der Dichtung immer ein sukzessives Öffnen und Schließen des Ventilsitzes gegeben.

In einer vorteilhaften Ausführung des Ventils nach der Erfindung wird vorgeschlagen, daß das schräg angeordnete Teil ein Teil einer spiralförmigen Scheibe ist, wobei die Scheibe am Ventilgehäuse befestigt ist. Hierdurch ist erreicht, daß die Scheibe und damit auch das schräg angeordnete Teil ein separates Teil ist, das bei Bedarf ausgewechselt werden kann.

Das schräg angeordnete Teil kann ein Teil einer Blattfeder sein, wobei die Blattfeder am Ventilgehäuse befestigt ist. Das schräg angeordnete Teil kann auch hier bei Bedarf ausgewechselt werden.

Das schräg angeordnete Teil weist nach der Erfindung zur Ebene der Scheibe bzw. zur Blattfeder einen Winkel auf, der zwischen 5° und 40°, vorzugsweise 30° liegt.

Die Erfindung ist nachfolgend anhand mehrerer in den Zeichnungen dargestellter Ausführungsbeispiele näher erläutert. Es zeigen:
- FIG. 1: eine Prinzipskizze eines Ventils nach der Erfindung mit einem Verschlußteil in einer Lage, in der die Ventilöffnung offen ist,
- FIG. 2 und 3: einen Teilschnitt eines Ventils nach FIG. 1 mit dem Verschlußteil in unterschiedlichen Lagen,
- FIG. 4: einen Teilschnitt eines Ventils nach FIG.1 mit einer anderen Ausführungsform des Verschlußteils als die, die in der FIG. 1 bis 3 dargestellt ist,
- FIG. 5: einen Teilschnitt eines Ventils nach FIG. 1 mit einer weiteren Ausführungsform des Verschlußteils,
- FIG. 6: eine Prinzipskizze eines Ventils nach der Erfindung mit noch einer Ausführungsform des Verschlußteils in einer Lage, in der die Ventilöffnung offen ist,
- FIG. 7: einen Teilschnitt eines Ventils nach FIG. 6 mit dem Verschlußteil in einer teilsweise offenen Lage,
- FIG. 8: eine Draufsicht einer Scheibe mit einem Teil, das in Verbindung mit dem in den FIG. 6 und 7 gezeigtem Verschlußteil vorgesehen ist,
- FIG. 9: eine Seitenansicht einer Scheibe nach FIG. 8,
- FIG. 10: eine Draufsicht einer weiteren Scheibe mit einem Teil, das in Verbindung mit dem in den FIG 6 und 7 gezeigten Verschlußteil vorgesehen ist,
- FIG. 11: eine Seitenansicht der Scheibe nach FIG. 10 und
- FIG. 12: ein Gasflußdiagramm

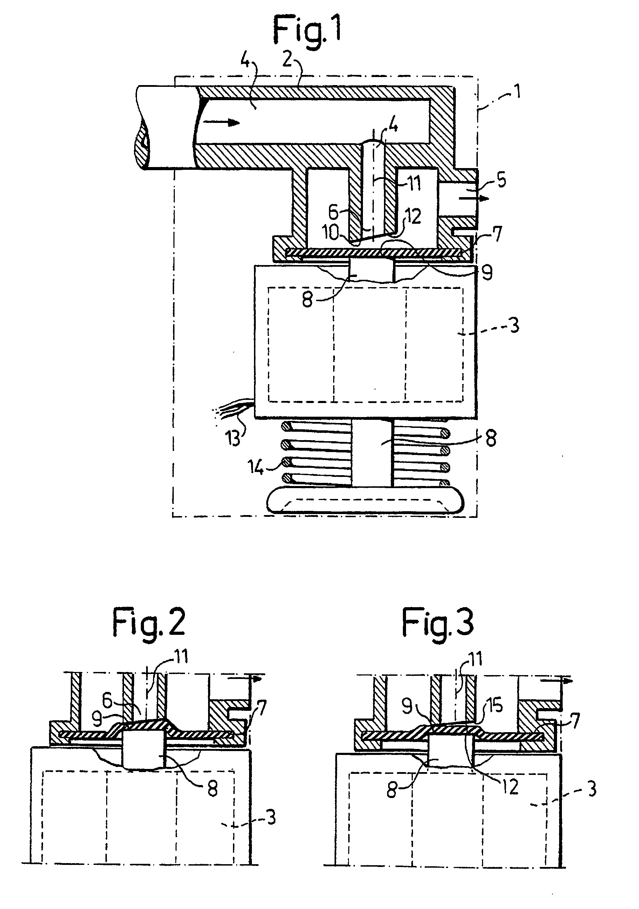

In der FIG. 1 ist, teils im Schnitt, eine mögliche Ausführungsform eines Ventils nach der Erfindung dargestellt. Das Ventil ist hier in einer offenen Lage gezeigt. Das Ventil weist eine Ventilumhüllung 1, in der ein Ventilgehäuse 2 und ein Solenoid 3 angeordnet sind, auf. Das Ventilgehäuse 2 ist mit einem durchgehenden Kanal für einen Gasfluß, der geregelt werden soll, versehen, wobei der Kanal in einen Einlaß 4, einen Auslaß 5 und eine dazwischen angeordnete Ventilöffnung 6 mit einem Ventilsitz 12 unterteilt ist. Genau gegenüber der Ventilöffnung 6 ist eine Membran 7 angebracht, die am Ventilgehäuse 2 festgespannt ist und die mit Hilfe der Endseite 9 der Solenoidenwelle 8 dazu dient, den Gasfluß zu einem Patienten in Verbindung mit einem hier nicht gezeigten Ventilator zu regeln. In der FIG. ist gezeigt, daß die Ebene 10 für den Ventilsitz 12 eine im Verhältnis zu seiner Zentrumachse 11 schräge Ausbildung hat, wobei die Ebene der Endseite 9 der Solenoidenwelle 8 und die Ebene 10 des Ventilsitzes 12 auf diese Weise einen Winkel bilden. Der Winkel zwischen den genannten Ebenen ist zwischen 0,3° und 2°, vorzugsweise 0,7°.

Um eine offene Ventillage, so wie es in der FIG 1 gezeigt ist, zu erreichen, wird dem Solenoid 3 mehr oder weniger Strom über ein Kabel 13 zugeführt. Die Welle 8 wird hierdurch in das Solenoidengehäuse hineingezogen, d.h. nach unten in dieser FIG 1, wobei die Membran 7 durch die Federkraft des Materials und durch den Gasdruck auf der Einlaßseite, auf gewünschte Weise von dem Ventilsitz 12 abhebt, so daß das Gas über die Ventilöffnung 6 und über den Auslaß 5 weiter zum Patienten strömen kann. Wenn die Stromzufuhr zum Solenoid 3 ausgeschaltet wird, wird dessen Welle 8 kraftlos, wobei die Welle 8 mit Hilfe einer Druckfeder 14 gegen die Membran 7 mit einer ausreichenden Kraft gegen den Ventilsitz 12 drückt, so daß die Ventilöffnung 6 wie es in der FIG 2 gezeigt ist, verschlossen wird. Hierdurch wird ein angestrebter Sicherheitsaspekt erzielt, daß nämlich bei einem Stromausfall das Ventil schließt und eine unkontrollierte Gasabgabe an den Patienten verhindert wird.

In der FIG 3 ist gezeigt, daß die Membran 7 beim Öffnen der Ventilöffnung 6 zunächst von demjenigen Teilbereich des Ventilsitzes 12, der den größten Abstand zur Endseite 9 der Solenoidenwelle 8 hat, abhebt. Hierdurch wird an diesem Teil zwischen dem Ventilsitz 12 und der Membran 7 im Öffnungsmoment immer ein Spalt 15 gebildet. Die Membran 7 kann nun durch die beschriebene Ausbildung des Ventilsitzes 12 die Ventilöffnung 6 sukzessiv öffnen. Beim Schließen der Ventilöffnung 6 wird die Membran 7 mit Hilfe des Wellenendes 9 sukzessiv gegen den Ventilsitz 12 gedrückt, wobei der Spalt 15 wieder auftritt, bevor die Membran 7 endgültig gegen den Ventilsitz 12 abdichtet.

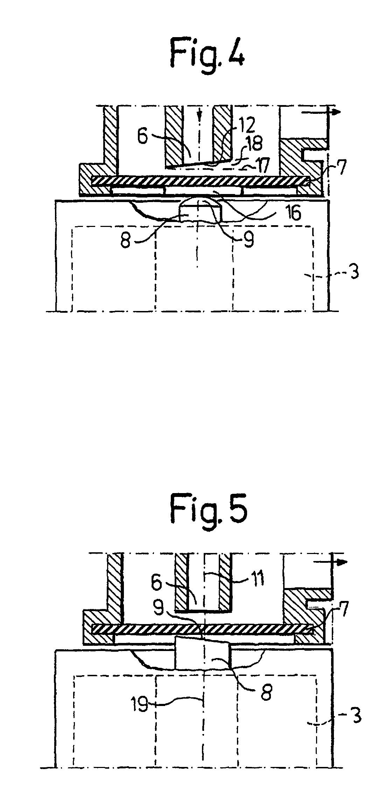

In einem weiteren in der FIG 4 gezeigten Ausführugsbeispiel ist die Seite der Membran 7, die dem Ventilsitz 12 abgewandt ist, mit einer steifen, ebenen Platte 16 versehen. Die Platte 16 weist eine Flächengröße auf, die etwas größer als die Flächengröße des Ventilsitzes 12 ist. In der FIG. ist gezeigt, daß das Ende 9 der Solenoidenwelle 8 gegen die Platte 16 anliegt, wobei das Wellenende 9 vorzugsweise abgerundet ausgebildet ist. Hier ist die Ventilöffnung 6 in einer offenen Lage gezeigt. Wenn die Membran 7 beim Schließen der Ventilöffnung 6 über das Wellenende 9 in Richtung des schräg vorgeformten Ventilsitzes 12 gedrückt wird, legt sich die Membran 7 zunächst gegen den Teilbereich des Ventilsitzes 12, der den kürzesten Abstand zur Membran 7 hat, an. Danach kippt die Platte 16 und damit die Membran 7 gegen das abgerundete Wellenende 9, so daß die Membran 7 sukzessiv dicht gegen den Ventilsitz 12 angelegt wird. Die strichpunktierten Linien 17 zwischen dem Ventilsitz 12 und der Membran 7 sollen dies verdeutlichen. Beim Öffnen der Ventilöffnung 6 kippt die Platte 16 und damit die Membran 7 gegen das abgerundete Wellenende 9, wobei die Membran 7 die Verbindung mit dem Ventilsitz 12 sukzessiv lösen kann. Sowohl im Schließungsmoment als auch im Öffnungsmoment entsteht ein Spalt 18 zwischen dem Ventilsitz 12 und der Membran 7. Die Platte 16 kann, wie es die strichpunktierten Linien andeuten, auch in der Membran 7 integriert sein.

In der FIG. 5 ist in einem weiteren Ausführungsbeispiel gezeigt, daß die Ebene der Endseite 9 der Solenoidenwelle 8 eine im Verhältnis zur Zentrumachse 19 schräge Ausbildung aufweist. In einem solchen Fall liegt die Ebene des Ventilsitzes 12 senkrecht zu seiner Zentrumachse 11. Das Vorgehen beim Öffnen und Schließen der Ventilöffnung 6 ist in etwa dasselbe wie bereits beschrieben, insbesondere in Verbindung mit den FIG. 1 bis 3.

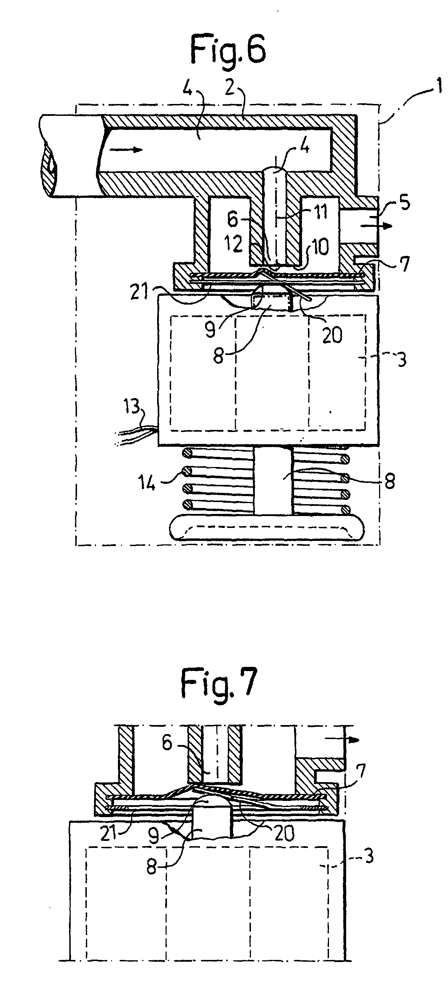

In der FIG. 6 ist ein Ventil, entsprechend dem Ventil, das in der FIG 1 gezeigt und in Verbindung hiermit näher beschrieben wurde, dargestellt. Der einzige Unterschied ist der, daß die Ebene 10 des Ventilsitzes 12 bei diesem Ventil im Verhältnis zu seiner Zentrumachse 11 senkrecht verläuft. Bei diesem Ventil ist zwischen der Endseite 9 der Solenoidenwelle 8 und dem Ventilsitz 12 ein gegenüber dem Ventilsitz 12 schräg liegendes Teil 20 angeordnet, wobei das Teil 20 ein Teil einer später näher beschriebenen spiralförmigen, im Ventilgehäuse 2 befestigten Scheibe 21 sein kann. Die Membran kann, wie es hier gezeigt ist, zwischen der Scheibe 21 und dem Ventilsitz 12 angebracht sein. Die Ebene des Wellenendes 9 kann, wie es mit den strichpunktierten Konturen gezeigt ist, parallel zu der Ebene 10 des Ventilsitzes 12 angeordnet sein, kann aber auch vorzugsweise abgerundet sein. Das Vorgehen beim Öffnen und Schließen des Ventilsitzes ist in etwa dasselbe wie es in Verbindung mit der FIG. 4 beschrieben worden ist und weist die Vorteile auf, die in Verbindung hiermit erhalten werden.

In der FIG. 6 ist die Ventilöffnung 6 in einer offenen Lage gezeigt. In der FIG. 7 ist die Ventilöffnung 6 in einer zur Hälfte offenen Lage gezeigt, bei der das Wellenende 9 mittels des Teils 20 die Membran 7 gegen einen Teilbereich des Ventilsitzes 12 drückt, um danach die Ventilöffnung 6 sukzessiv zu schließen. In den Öffnungs- und Schließmomenten entsteht wie bereits beschrieben ein Spalt zwischen der Membran 7 und dem Ventilsitz 12.

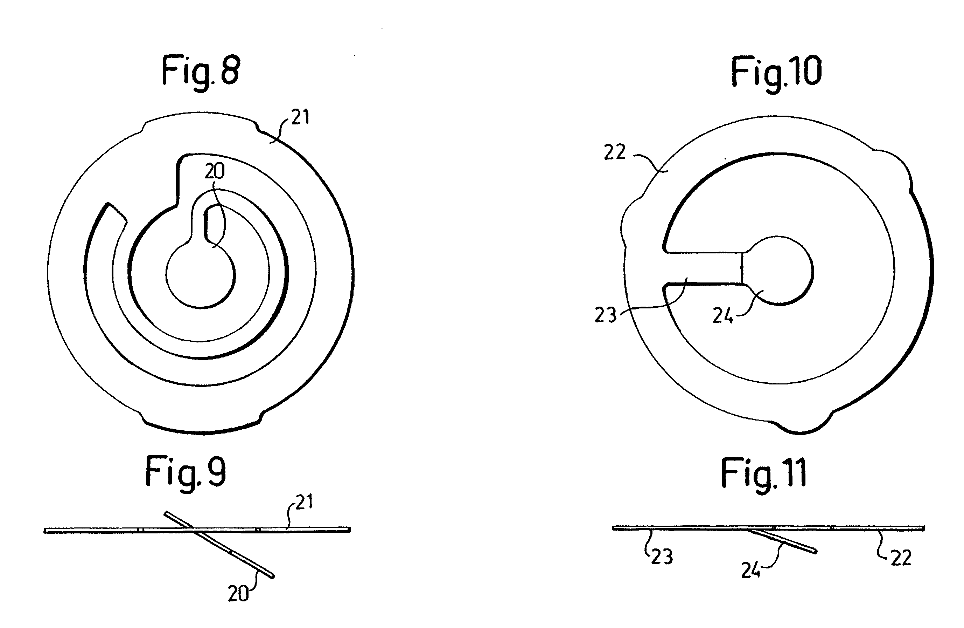

In der FIG. 8 ist eine Draufsicht einer Scheibe 21 mit dem schräg geformten Teil 20 gezeigt. Die Scheibe 21 besteht aus einem solchen Material, daß sie in allen Lagen danach strebt, die gezeigte schräge Lage einzunehmen.

In der FIG. 9 ist eine Seitenansicht der Scheibe 21 gezeigt. Das schräg gestellte Teil 20 weist im Vergleich zur Ebene der Scheibe 21 einen Winkel von zwischen 5° und 40°, vorzugsweise 30° auf.

In den FIG. 10 und 11 ist eine alternative Scheibe 22 mit einer Blattfeder 23 und einem schräg angeordneten Teil 24 gezeigt.

Die Dichtung ist bis jetzt in Form einer Membran, die im Ventilgehäuse befestigt ist, beschrieben worden. Im Rahmen der Erfindung kann in den Fällen, wo es möglich ist, statt einer Membran eine Dichtung verwendet werden, die vorzugsweise am Ventilsitz, an der Endseite der Solenoidenwelle oder an dem schräg angeordneten Teil appliziert wird. Die Membran kann auch mit den zuletzt erwähnten Dichtungen kombiniert werden. In solchen Fällen ist die Membran lediglich eine bewegliche Trennwand zwischen dem Solenoid und dem Ventilgehäuse, die verhindert, daß Gas in das Solenoid hineindringt.

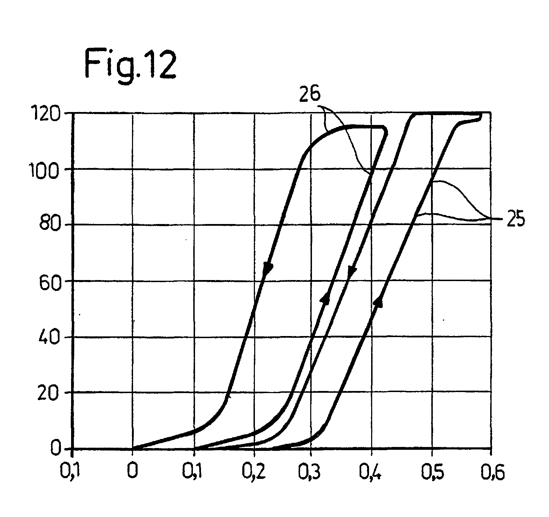

In der FIG. 12 sind Gasflüsse graphisch wiedergegeben, wobei der Gasfluß/Min. entlang der vertikalen Achse und die Spannung, die der Solenoidenwelle 8 zugeführt wird, entlang der horizontalen Achse gezeigt worden sind. Die rechte Flußkurve 25 beschreibt einen Flußverlauf eines Ventils nach dem Stand der Technik und die linke Flußkurve 26 beschreibt einen Flußverlauf eines Ventils nach der Erfindung. Ein scharfer Knick an der steigenden Kurve der rechten Flußkurve 25 zeigt, daß ein verhältnismäßig abruptes Öffnen der Ventilöffnung erfolgt. Auch die sinkende Kurve zeigt durch einen scharfen Knick, daß ein abruptes Schließen der Ventilöffnung erfolgt. Die Flußkurve 25 zeigt damit die Probleme in Verbindung mit einem bekannten Ventil, insbesondere kleine Flüsse mit einer hohen Genauigkeit und Wiederholbarkeit zu regeln. Die linke Flußkurve 26 zeigt, wie ein sukzessives Öffnen und Schließen der Ventilöffnung bei einem Ventil nach der Erfindung die Kurvenform verändern kann. Die Kurvenform verläuft nunmehr kontinuierlich, wobei ein verhältnismäßig weicher Übergang von einer geschlossenen in eine offene Lage und umgekehrt erreicht worden ist.

Durch das beschriebene sukzessive Öffnen und Schließen der Ventilöffnung, das mit dem bereits beschriebenen Spalt zwischen dem Ventilsitz und der Dichtung beginnt bzw. endet, können insbesondere kleine Gasmengen mit einer großen Genauigkeit und Wiederholbarkeit und mit einer großen Dynamik geregelt werden.

## Patentansprüche

1. Ventil zum Regeln eines Gasflusses, umfassend ein Ventilgehäuse (2) mit einem Einlaß (4) für das Gas, das geregelt werden soll, und einem Auslaß (5) für einen geregelten Gasfluß, einem Ventilsitz (12) mit einer zwischen dem Einlaß (4) und dem Auslaß (5) angeordneten Ventilöffnung (6), einem bewegbaren Verschlußteil (8), umfassend eine steuerbare Welle (7), wobei die Endseite (9) des Verschlußteils (8) eine Dichtung (7), die zwischen dem Ventilsitz (12) und dem Verschlußteil (8) angeordnet ist, derart beeinflussen kann, daß das Verschlußteil (8) die Ventilöffnung (6) schließt und öffnet sowie den Gasfluß durch die Ventilöffnung (6) regelt, **dadurch gekennzeichnet, daß** die Ebene der Endseite (9) des Verschlußteils (8) und die Ebene (10) des Ventilsitzes (12) einen Winkel bilden, wobei die Endseite (9) des Verschlußteils (8) derart gegen den Ventilsitz (12) gedrückt bzw. von diesem gelöst werden kann, daß die Endseite (9) die Ventilöffnung (6) sukzessiv schließt bzw. öffnet.

2. Ventil nach Anspruch 1, **dadurch gekennzeichnet, daß** die Ebene (10) des Ventilsitzes (12) schräg zur Zentrumachse (11) des Ventilsitzes (12) liegt.

3. Ventil nach Anspruch 1, **dadurch gekennzeichnet, daß** die Ebene der Endseite (9) des Verschlußteils (8) schräg zu dessen Zentrumachse (19) liegt.

4. Ventil nach einem der Ansprüche 1 bis 3, **dadurch gekennzeichnet, daß** der Winkel zwischen den genannten Ebenen (9, 10) zwischen 0,3° und 2°, vorzugsweise 0,7° beträgt.

5. Ventil nach einem der Ansprüche 1 bis 4, **dadurch gekennzeichnet, daß** die Dichtung eine am Ventilgehäuse (2) festgespannte Membran (7) ist und daß die Membran (7) mit einer steifen ebenen Platte (16) versehen ist, die genau vor dem Ventilsitz (12) angeordnet ist und die eine Flächengröße aufweist, die wenigstens die Flächengröße des Ventilsitzes (12) hat und daß der Teil der Endseite (9) des Verschlußteils (8), der die Membran (7) beeinflußt, eine Flächengröße aufweist, die kleiner als die Flächengröße des Ventilsitzes (8) ist.

6. Ventil nach Anspruch 5, **dadurch gekennzeichnet, daß** die Endseite (9) des Verschlußteils (8) abgerundet ausgebildet ist.

7. Ventil zum Regeln eines Gasflusses, umfassend ein Ventilgehäuse (2) mit einem Einlaß (4) für das Gas, das geregelt werden soll und einem Auslaß (5) für einen geregelten Gasfluß, einem Ventilsitz (12) mit einer zwischen dem Einlaß (4) und dem Auslaß (5) angeordneten Ventilöffnung (6), einem bewegbaren Verschlußteil (8), umfassend eine steuerbare Welle (8), wobei die Endseite (9) des Verschlußteils (8) eine Dichtung (7), die zwischen dem Ventilsitz (12) und dem Verschlußteil (8) angeordnet ist, derart beeinflussen kann, daß das Verschlußteil (8) die Ventilöffnung (6) schließt und öffnet sowie den Gasfluß durch die Ventilöffnung (6) regelt, **dadurch gekennzeichnet, daß** zwischen der Endseite (9) des Verschlußteils (8) und dem Ventilsitz (12) ein gegenüber dem Ventilsitz (12) schräg angeordnetes, vorgeformtes Teil (20, 24) angebracht ist, das eine Flächengröße aufweist, die wenigstens der Flächengröße des Ventilsitzes (12) entspricht, wobei die Endseite (9) des Verschlußteils (8) so angeordnet ist, daß sie das schräg angeordnete Teil (20, 24) derart gegen den Ventilsitz (12) drückt bzw. vom Ventilsitz (12) löst, daß das schräg angeordnete Teil (20, 24) die Ventilöffnung (6) sukzessiv schließt bzw. öffnet.

8. Ventil nach Anspruch 7, **dadurch gekennzeichnet, daß** der Teil der Endseite (9) des Verschlußteils (8), der das schräg angeordnete Teil (20, 24) beeinflußt, eine Flächengröße aufweist, die kleiner als die Flächengröße des Ventilsitzes (12) ist.

9. Ventil nach Anspruch 7 oder 8, **dadurch gekennzeichnet, daß** die Endseite (9) des Verschlußteils (8) abgerundet ausgebildet ist.

10. Ventil nach Anspruch 7 oder 8, **dadurch gekennzeichnet, daß** die Ebene der Endseite (9) des Verschlußteils (8) und die Ebene (10) des Ventilsitzes (12) parallel zueinander angeordnet sind.

11. Ventil nach einem der Ansprüche 7 bis 10, **dadurch gekennzeichnet,daß** das schräg angeordnete Teil (20) ein Teil einer spiralförmigen Scheibe (21) ist, wobei die Scheibe 21 am Ventilgehäuse (2) befestigt ist.

12. Ventil nach einem der Ansprüche 7 bis 10, **dadurch gekennzeichnet, daß** das schräg angeordnete Teil (24) ein Teil einer Blattfeder (23) ist, wobei die Blattfeder (23) am Ventilgehäuse (2) befestigt ist.

13. Ventil nach einem der Ansprüche 7 bis 12, **dadurch gekennzeichnet, daß** das schräg angeordnete Teil (20, 24) zur Ebene der Scheibe (21) bzw. der Blattfeder (23) einen Winkel aufweist, der zwischen 5° und 40°, vorzugsweise 30° liegt.

## Claims

1. Valve for regulating a gas flow, comprising a valve housing (2) with an inlet (4) for the gas, which is to be regulated and an outlet (5) for a regulated gas flow, with a valve seat (12) having a valve orifice (6) arranged between the inlet (4) and the outlet (5), and with a movable closure part (8) comprising a controllable shaft (7), the end face (9) of the closure part (8) being capable of influencing a seal (7) arranged between the valve seat (12) and the closure part (8), in such a way that the closure part (8) closes and opens the valve orifice (6) and regulates the gas flow through the valve orifice (6), **characterized in that** the plane of the end face (9) of the closure part (8) and the plane (10) of the valve seat (12) form an angle, the end face (9) of the closure part (8) being capable of being pressed against the valve seat (12) or released from the latter in such a way that the end face (9) successively closes and opens the valve orifice (6).

2. Valve according to Claim 1, **characterized in that** the plane (10) of the valve seat (12) lies obliquely to the centre axis (11) of the valve seat (12).

3. Valve according to Claim 1, **characterized in that** the plane of the end face (9) of the closure part (8) lies obliquely to the centre axis (19) of the latter.

4. Valve according to one of Claims 1 to 3, **characterized in that** the angle between the said planes (9, 10) is between 0.3° and 2°, preferably 0.7°.

5. Valve according to one of Claims 1 to 4, **characterized in that** the seal is a diaphragm (7) firmly clamped to the valve housing (2), and **in that** the diaphragm (7) is provided with a rigid planar plate (16) which is arranged exactly in front of the valve seat (12) and which has a surface size having at least the surface size of the valve seat (12), and **in that** that part of the end face (9) of the closure part (8) which influences the diaphragm (7) has a surface size which is smaller than the surface size of the valve seat (8).

6. Valve according to Claim 5, **characterized in that** the end face (9) of the closure part (8) is of rounded design.

7. Valve for regulating a gas flow, comprising a valve housing (2) with an inlet (4) for the gas which is to be regulated and an outlet (5) for a regulated gas flow, with a valve seat (12) having a valve orifice (6) arranged between the inlet (4) and the outlet (5), and with a movable closure part (8) comprising a controllable shaft (8), the end face (9) of the closure part (8) being capable of influencing a seal (7) arranged between the valve seat (12) and the closure part (8), in such a way that the closure part (8) closes and opens the valve orifice (6) and regulates the gas flow through the valve orifice (6),
**characterized in that** between the end face (9) of the closure part (8) and the valve seat (12) is mounted a preformed part (20, 24) which is arranged obliquely with respect to the valve seat (12) and which has a surface size corresponding at least to the surface size of the valve seat (12), the end face (9) of the closure part (8) being arranged in such a way that the said end face presses the obliquely arranged part (20, 24) against the valve seat (12) or releases it from the valve seat (12) in such a way that the obliquely arranged part (20, 24) successively closes and opens the valve orifice (6).

8. Valve according to Claim 7, **characterized in that** that part of the end face (9) of the closure part (8) which influences the obliquely arranged part (20, 24) has a surface size which is smaller than the surface size of the valve seat (12).

9. Valve according to Claim 7 or 8, **characterized in that** the end face (9) of the closure part (8) is of rounded design.

10. Valve according to Claim 7 or 8, **characterized in that** the plane of the end face (9) of the closure part (8) and the plane (10) of the valve seat (12) are arranged parallel to one another.

11. Valve according to one of Claims 7 to 10, **characterized in that** the obliquely arranged part (20) is part of a spiral disc (21), the disc (21) being fastened to the valve housing (2).

12. Valve according to one of Claims 7 to 10, **characterized in that** the obliquely arranged part (24) is part of a leaf spring (23), the leaf spring (23) being fastened to the valve housing (2).

13. Valve according to one of Claims 7 to 12, **characterized in that** the obliquely arranged part (20, 24) is at an angle to the plane of the disc (21) or of the leaf spring (23) which is between 5° and 40°, preferably 30°.

## Revendications

1. Vanne de réglage d'un courant de gaz, comprenant un corps (2) de vanne ayant une entrée (4) pour le gaz qui doit être réglé et une sortie (5) de gaz réglé, un siège (12) de vanne ayant une ouverture (6) de vanne interposée entre l'entrée (4) et la sortie (5), une partie (8) mobile formant obturateur et comprenant un arbre (7) qui peut être commandé, le côté (9) d'extrémité de la partie (8) formant obturateur pouvant influer sur une garniture (7) d'étanchéité interposée entre le siège (12) de vanne et la partie (8) formant obturateur, de façon à ce que la partie (8) formant obturateur ferme et ouvre l'ouverture (6) de la vanne ainsi que règle le courant de gaz passant par l'ouverture (6) de la vanne, **caractérisée en ce que** le plan du côté (9) d'extrémité de la partie (8) de fermeture et le plan (10) du siège (12) de la vanne ont un angle, le côté (9) d'extrémité de la partie (8) formant obturateur pouvant être repoussé sur le siège (12) de la vanne ou pouvant en être détaché, de façon à ce que le côté (9) d'extrémité ferme et ouvre successivement l'ouverture (6) de la vanne.

2. Vanne suivant la revendication 1, **caractérisée en ce que** le plan (10) du siège (12) de la vanne est incliné par rapport à l'axe (11) de centre du siège (12) de la vanne.

3. Vanne suivant la revendication 1, **caractérisée en ce que** le plan du côté (9) d'extrémité de la partie (8) formant obturateur est incliné par rapport à son axe (19) de centre.

4. Vanne suivant l'une des revendications 1 à 3, **caractérisée en ce que** l'angle compris entre les plans (9, 10) mentionnés est compris entre 0,3° et 2° et de préférence est de 0,7°.

5. Vanne suivant l'une des revendications 1 à 4, **caractérisée en ce que** la garniture d'étanchéité est une membrane (7) serrée sur le corps (2) de la vanne et **en ce que** la membrane (7) est munie d'un plateau (16) rigide et plan qui est disposé exactement devant le siège (12) de la vanne et qui a une dimension en surface ayant au moins la dimension en surface du siège (12) de la vanne et **en ce que** la partie du côté (9) d'extrémité de la partie (8) formant obturateur qui influe sur la membrane (7) a une dimension en surface qui est inférieure à la dimension en surface du siège (8) de la vanne.

6. Vanne suivant la revendication 5, **caractérisée en ce que** le côté (9) d'extrémité de la partie (8) formant obturateur est arrondi.

7. Vanne de réglage d'un courant de gaz, comprenant un corps (2) de vanne ayant une entrée (4) pour le gaz qui doit être réglé et une sortie (5) de gaz réglé, un siège (12) de vanne ayant une ouverture (6) de vanne interposée entre l'entrée (4) et la sortie (5), une partie (8) mobile formant obturateur et comprenant un arbre (7) qui peut être commandé, le côté (9) d'extrémité de la partie (8) formant obturateur pouvant influer sur une garniture (7) d'étanchéité interposée entre le siège (12) de vanne et la partie (8) formant obturateur, de façon à ce que la partie (8) formant obturateur ferme et ouvre l'ouverture (6) de la vanne ainsi que règle le courant de gaz passant par l'ouverture (6) de la vanne, **caractérisée en ce qu'**il est prévu entre le côté (9) d'extrémité de la partie (8) formant obturateur et le siège (12) de la vanne une partie (20, 24) qui est inclinée par rapport au siège (12) de la vanne qui est préformé et qui a une dimension en surface qui correspond au moins à la dimension en surface du siège (12) de la vanne, le côté (9) d'extrémité de la partie (8) formant obturateur étant disposé de façon à ce qu'il repousse sur le siège (12) de la vanne ou écarte du siège (12) de la vanne la partie (20, 24) disposée de manière inclinée, de façon à ce que la partie (20, 24) disposée de manière inclinée ouvre et ferme successivement l'ouverture (6) de la vanne.

8. Vanne suivant la revendication 7, **caractérisée en ce que** la partie du côté (9) d'extrémité de la partie (8) formant obturateur qui influe sur la partie (20, 24) disposée de manière inclinée a une dimension en surface qui est inférieure à la dimension en surface du siège (12) de la vanne.

9. Vanne suivant la revendication 7 ou 8, **caractérisée en ce que** le côté (9) d'extrémité de la partie (8) formant obturateur est arrondi.

10. Vanne suivant la revendication 7 ou 8, **caractérisée en ce que** le plan du côté (9) d'extrémité de la partie (8) formant obturateur et le plan (10) du siège (12) de la vanne sont parallèles.

11. Vanne suivant l'une des revendications 7 à 10, **caractérisée en ce que** la partie (20) disposée de manière inclinée est une partie d'un disque (21) en forme de spirale, le disque (21) étant fixé au corps (2) de la vanne.

12. Vanne suivant l'une des revendications 7 à 10, **caractérisée en ce que** la partie (24) disposée de manière inclinée est une partie d'un ressort (23) à lame, le ressort (23) à lame étant fixé au corps (2) de la vanne.

13. Vanne suivant l'une des revendications 7 à 12, **caractérisée en ce que** la partie (20, 24) disposée de manière inclinée fait avec le plan du disque (21) ou du ressort (23) à lame un angle qui est compris entre 5° et 40° et qui est de préférence de 30°.
